# EUROPEAN PATENT SPECIFICATION

(11) **EP 2 478 834 B1**
(45) Date of publication and mention of the grant of the patent: **24.06.2015**
(21) Application number: 10816884.0
(22) Date of filing: 15.09.2010
(51) Int. Cl.: A61B 5/00, G06T 1/00

(54) **RADIOGRAPH INTERPRETATION REPORT CREATION DEVICE, METHOD, AND PROGRAM**
VORRICHTUNG, VERFAHREN UND PROGRAMM ZUR ERSTELLUNG VON RÖNTGENBILD-INTERPRETATIONSBERICHTEN
DISPOSITIF DE CRÉATION DE RAPPORTS D'INTERPRÉTATION DE RADIOGRAPHIES, PROCÉDÉ ET PROGRAMME ASSOCIÉS

(30) Priority: 17.09.2009 JP 2009215579; 23.03.2010 JP 2010066566
(43) Date of publication of application: 25.07.2012
(73) Proprietor: FUJIFILM Corporation, Tokyo 106-8620 (JP)
(72) Inventor: MORIYA, Yoshiyuki, Tokyo 107-0052 (JP)
(74) Representative: Klunker . Schmitt-Nilson . Hirsch
(86) International application number: PCT/JP2010/005640
(87) International publication number: WO 2011/033770

(56) References cited:
- WO-A1-2009/073185
- JP-A- 2007 012 044
- JP-A- 2007 264 773
- JP-A- 2007 275 558
- US-A1- 2007 239 489

## Description

### BACKGROUND OF THE INVENTION

### Field of the Invention

The present invention relates to an image interpretation report generation apparatus according to the preamble of claim 1, and an image interpretation report generation method and computer program.

### Description of the Related Art

In recent years, in the field of medical treatment, doctors specialized in image-based diagnosis read medical images, and record the results of reading, as image interpretation reports, and report the results to doctors in charge of patients. Further, electronic image interpretation reports are widely used as the image interpretation reports.

As such image interpretation reports, Japanese Unexamined Patent Publication No. 2005-301453 (Patent Document 1) discloses a technique that can generate an image interpretation report including a character string linked, by a hyperlink, to a reference image. Such an image interpretation report is used, and a person, such a doctor in charge, who reads an image can display and observe a corresponding reference image by clicking a character in the image interpretation report on a display screen. Therefore, it is possible to more easily understand the image interpretation report.

However, generation of an image interpretation report with a hyperlink, as described above, requires a linking operation in addition to a conventional operation for generating an image interpretation report. In the linking operation, a character string in the image interpretation report to be generated is selected, and a reference image is dragged and dropped to the position of the selected character string. Therefore, there has been a problem that a work load in input of the image interpretation report increases.

Further, in Patent Document 1, a character string is linked to an image. Therefore, when plural link target regions are present in a slice image, only the same corresponding medical image is observable, irrespective of selection of any link character from the plural linked character strings. Hence, there has been a problem that it is impossible to recognize a position in the medical image indicated by each character string.

To solve this problem, a method in which new plural reference images are generated by cutting out, from a slice image, each of plural linked target regions, and in which linking operations are performed by dragging and dropping the generated reference images to corresponding character strings, respectively, was proposed. However, the method had a problem that the edit operation is extremely complicated.

In accordance with the preamble of claim 1, WO-A-2009/073185 discloses an image interpretation report generation apparatus in which, based on the displayed medical image, a similar reference image is retrieved from a storage. One or more substantial anatomical features or structures of the body portion shown in the medical image are segmented. Specific features can be illustrated in a specific (coloured or shaded) manner based on data from the reference image and associated data. The user can click an anatomical feature or structure and thereby causing that text descriptions on the display related to the feature or structure are shown or are hidden.

US 2007/0239489 A1 discloses an imaging diagnosis supporting apparatus in addition to an image based on normal image data. A shared object is constructed by supplementary information. Such shared object is generated as an entity of information separated from normal image data.

### SUMMARY OF THE INVENTION

In view of the foregoing circumstances, it is an object of the present invention to provide an image interpretation apparatus and method that can make it possible to more easily generate an image interpretation report including a hyperlink, and that can make it possible to more easily understand a medical image corresponding to a linked character string.

An image interpretation report generation apparatus of the present invention is an image interpretation report generation apparatus comprising the features of claim 1.

An image interpretation report generation method of the present invention is an image interpretation report generation method comprising the steps of:
obtaining and displaying a medical image;
generating and displaying an electronic image interpretation report of the medical image;
inputting the position of a lesion region in the medical image;
inserting, into the image interpretation report, a link character linked by a hyperlink to the medical image including the input position of the lesion region in such a manner that the link character is distinguishably displayed;
selecting the distinguishably displayed link character in the image interpretation report; and
displaying an index representing the position of the lesion region in the medical image based on the selection of the link character.

An image interpretation report generation program of the present invention is an image interpretation report generation program for causing a computer to function as an apparatus defined by claim 1.

Here, the "position of a lesion region" may be a point representing an arbitrary position in a lesion region, or the lesion region, itself. A region may be represented by using various methods, such as a circle, a rectangle, an arrow, and a closed curve.

Further, "input of the position of a lesion region" may be performed by directly inputting the position of a lesion by using a mouse, a keyboard, or other input devices. Alternatively, the position of a lesion region calculated based on a coordinate input by a mouse, a keyboard or the like may be input. Further, the position of a lesion region that has been recognized by using a known image recognition function for automatically detecting an abnormal shadow region in a medical image by image processing may be input.

It is desirable that the "link character" linked by a hyperlink is a character string, a sign or a number that has been prepared in advance. Alternatively, the link character may be input by an operator.

The expression "inserts, into the image interpretation report, a link character in such a manner that the link character is distinguishably displayed" means that the link character is displayed distinguishably from the other characters so that the link character is outstanding in the image interpretation report. For example, the link character is displayed distinguishably from non-linked characters by using known methods, such as coloring the link character or the background of the link character, an underline, blinking, bold letters, a letter type, the size of the character, and a frame. Further, the link character inserted into the image interpretation report may be edited by changing a character string that has been prepared in advance or the like to a different character string or the like, while the linked state of the link character is maintained.

The term "selection of a link character" means selecting a link character by a mouse, a keyboard or other input devices.

In the image interpretation report generation apparatus of the present invention, the lesion region input means may be able to input the positions of a plurality of lesion regions, and the link character insertion means may be able to insert a plurality of link characters corresponding to the input positions of the plurality of lesion regions, respectively.

In the image interpretation report generation apparatus of the present invention, it is desirable that the image interpretation report display means displays, together with the image interpretation report, an attachment image obtained by reducing the medical image including the lesion region corresponding to the link character.

In the image interpretation report generation apparatus of the present invention, the image interpretation report display means may display a plurality of attachment images in the order of arrangement of the link characters in the image interpretation report.

In the image interpretation report generation apparatus of the present invention, the lesion region input means may be able to manually input the position of the lesion region. The expression "able to manually input the position of the lesion region" means that an operator can input the position of the lesion region by using an input device, such as a mouse and a keyboard.

It is desirable that the image interpretation report generation apparatus of the present invention further includes a lesion name storage means that stores lesion names representing a plurality of lesions, and a lesion region detection means that automatically detects the position of the lesion region in the medical image. Further, it is desirable that the lesion region input means inputs the position of the lesion region that has been automatically detected by the lesion region detection means, and that the link character insertion means selects, from the lesion name storage means, a lesion name representing the lesion present at the position of the lesion region that has been detected by the lesion region detection means, and inserts the lesion name.

The "lesion name" includes at least one of a disease name, a keyword or a sign representing the disease name, and abbreviations thereof. The lesion name may include the name of an organ or a keyword representing an organ.

As the "storage means", a hard disk and a recording medium, such as a flash memory, are appropriately used.

The expression "automatically detects the position of the lesion region" means detecting an abnormal shadow region including a lesion by using a known image recognition function for detecting an abnormal shadow in a medical image by image processing. The automatically detected lesion region may be an organ region.

The index display means may display the index, based on the selection of the link character in the image interpretation report, in such a manner that only the index corresponding to the selected link character is emphasized.

The link character insertion means may insert the plurality of link characters in different colors from each other into the image interpretation report, and the index display means may display the plurality of indices in the same colors as the colors of the link characters corresponding to the plurality of indices, respectively.

According to an image interpretation report generation apparatus, method and program of the present invention, a link character linked by a hyperlink to a medical image including the input position of the lesion region in the medical image is inserted into the image interpretation report in such a manner that the link character is distinguishably displayed. Therefore, a linking operation by a hyperlink is completed only by inputting a lesion region, and a complicated linking operation is not necessary. Hence, it is possible to more easily generate an image interpretation report including a hyperlink. Further, it is possible to display an index representing the position of a lesion region in a medical image by selecting a link character that is distinguishably displayed in the image interpretation report. Therefore, it is possible to more easily understand the medical image corresponding to the linked character string.

When the lesion region input means can input the positions of a plurality of lesion regions, and the link character insertion means can insert a plurality of link characters corresponding to the input positions of the plurality of lesion regions, respectively, plural corresponding link operations are easy. Therefore, it is not necessary to perform complicated linking operations, and it is possible to more easily generate an image interpretation report including a hyperlink.

When the image interpretation report display means of the present invention displays, together with the image interpretation report, an attachment image obtained by reducing the medical image including the lesion region corresponding to the link character, it is possible to easily refer to the attachment image of the linked image. Therefore, it is possible to more easily understand the medical image corresponding to the linked character string.

When the image interpretation report display means of the present invention displays a plurality of attachment images in the order of arrangement of the link characters in the image interpretation report, it is possible to easily refer to the attachment images of the linked images. Therefore, it is possible to more easily understand the medical images corresponding to the linked character strings.

When the lesion region input means of the present invention can manually input the position of the lesion region, a linking operation by a hyperlink is completed only by manually inputting the position of a lesion region, and a complicated linking operation is not necessary. Therefore, it is possible to more easily generate an image interpretation report including a hyperlink.

When the image interpretation report generation apparatus, method and program of the present invention further includes a lesion name storage means that stores lesion names representing a plurality of lesions, and a lesion region detection means that automatically detects the position of the lesion region in the medical image, and the lesion region input means inputs the position of the lesion region that has been automatically detected by the lesion region detection means, and the link character insertion means selects, from the lesion name storage means, a lesion name representing the lesion present at the position of the lesion region that has been detected by the lesion region detection means, and inserts the lesion name, a link character representing the position of a lesion region is automatically inserted into the image interpretation report. Therefore, it is possible to more easily generate an image interpretation report including a hyperlink.

When the index display means of the present invention displays the index, based on the selection of the link character in the image interpretation report, in such a manner that only the index corresponding to the selected link character is emphasized, it is possible to more easily understand the medical image corresponding to the linked character string.

When the link character insertion means of the present invention inserts the plurality of link characters in different colors from each other into the image interpretation report, and the index display means displays the plurality of indices in the same colors as the colors of the link characters corresponding to the plurality of indices, respectively, it is possible to easily understand correspondence between the plurality of link characters in the image interpretation report and the positions of the plurality of lesion regions. Therefore, it is possible to more easily understand the medical images corresponding to the linked character strings.

### BRIEF DESCRIPTION OF THE DRAWINGS

Figure 1 is a schematic diagram illustrating the configuration of an image interpretation report generation apparatus according to an embodiment of the present invention;
Figure 2 is a block diagram illustrating a configuration according to a first embodiment;
Figure 3 is a functional block diagram illustrating an image interpretation report generation function according to the first embodiment;
Figure 4 is a flowchart for explaining a flow of link character insertion processing in the first embodiment;
Figure 5 is a conceptual diagram of an image interpretation report and a medical image displayed by the image interpretation report generation function of the first embodiment;
Figure 6 is a flowchart for explaining a flow of link character change in the first embodiment;
Figure 7 is a conceptual diagram of an image interpretation report generated by changing a part of the link character in the first embodiment;
Figure 8 is a conceptual diagram of an image interpretation report and a medical image displayed in a second embodiment;
Figure 9 is a conceptual diagram of an image interpretation report and a medical image displayed in a modified example of the second embodiment;
Figure 10 is a functional block diagram illustrating an image interpretation report generation function according to a third embodiment;
Figure 11 is a flowchart for explaining a flow of link character insertion processing in the third embodiment;
Figure 12 is a diagram illustrating an example of a correspondence table showing correspondence between the positions of lesion regions and link character candidates; and
Figure 13 is a diagram illustrating an example of display of a candidate list of link characters.

### DESCRIPTION OF THE PREFERRED EMBODIMENTS

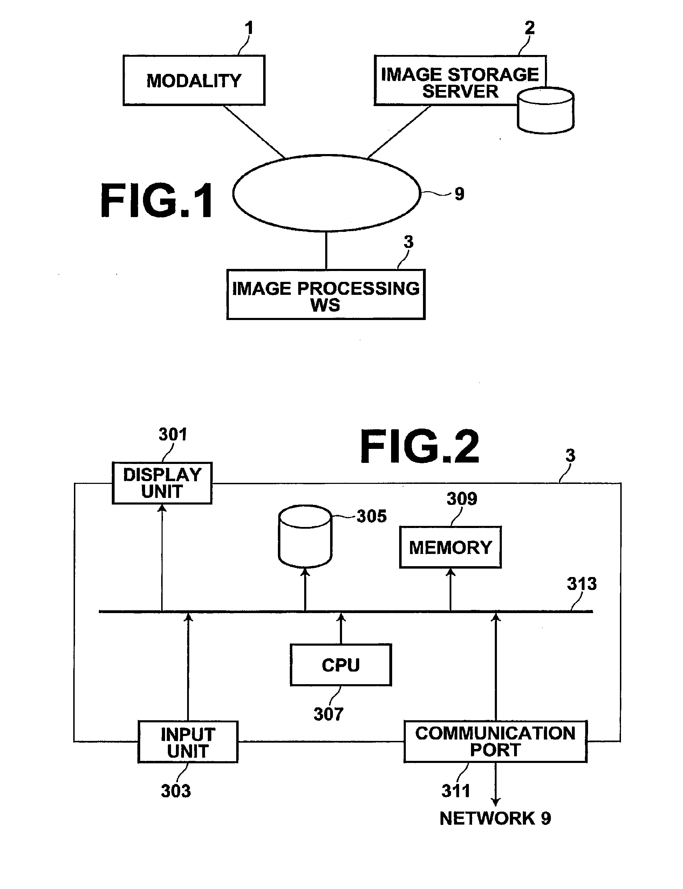

Figure 1 is a schematic diagram illustrating the hardware configuration of a medical image processing apparatus. As illustrated in Figure 1, in this system, a modality 1, an image storage server 2, and an image processing workstation 3 are connected to each other through a network 9 in such a manner that communication is possible therebetween.

The modality 1 obtains medical image V representing an object to be examined. Specifically, the modality 1 is a CT apparatus, an MRI apparatus, a PET, an ultrasonic diagnosis apparatus, and the like.

The image storage server 2 is a computer that stores, in an image database, medical image V obtained by the modality 1 and medical image V generated by image processing at the image processing workstation 3, and manages the medical image V. The image storage server 2 includes a large-capacity external recording device, and database management software (for example, ORDB (Object Relational Database) management software).

The image processing workstation 3 is a computer that performs, based on a request from a person who reads the image, image processing on medical image V obtained by the modality 1, or medical image V obtained from the image storage server 2, and displays a generated image.

The storage format of image data and communication between apparatuses through the network 9 are based on a protocol, such as DICOM (Digital Imaging and Communications in Medicine).

Next, a configuration related to a medical image processing function of the first embodiment will be described.

Figure 2 is a schematic block diagram illustrating the configuration of the image processing workstation 3. As illustrated in Figure 2, an image interpretation report generation apparatus of the first embodiment is constituted by the image processing workstation 3 including a display unit 301, such as a liquid crystal monitor, an input unit 303, a hard disk 305, a CPU 307, a memory 309, and a communication interface 311. The display unit 301 displays various kinds of information. The input unit 303 is composed of a keyboard, a mouse and the like for inputting various kinds of information. The hard disk 305 stores various programs for controlling the image interpretation report generation apparatus of the present embodiment and various kinds of data, such as image data. The various programs include an image interpretation report generation program of the present invention. The CPU 307 controls the image interpretation report generation apparatus according to the present embodiment by executing various programs. The memory 309 is a working area during execution of programs, and the communication interface 311 is connected to a network through a bus 313.

In all examples of the present invention, the function of the present invention is carried out by a computer by using a program installed from the outside. A set of information including the program may be provided by a recording medium, such as a CD-ROM, a flash memory, and FD, or from an external recording medium through a network, and installed.

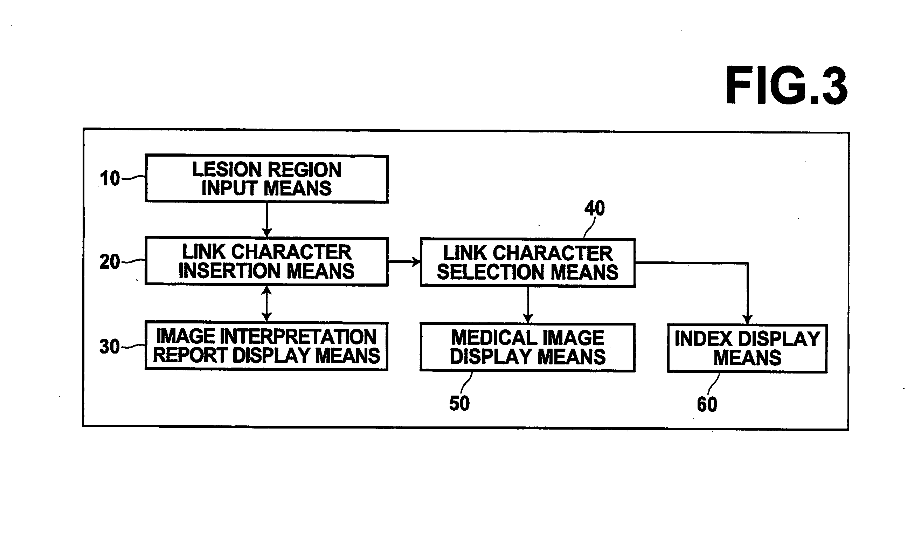

Figure 3 is a functional block diagram illustrating a part related to an image interpretation report generation function in the first embodiment of the present invention. As illustrated in Figure 3, the image interpretation report generation function of the present invention is composed of a medical image display means 50, an image interpretation report display means 30, a lesion region input means 10, a link character insertion means 20, a link character selection means 40, and an index display means 60. The medical image display means 50 obtains, based on a request from the modality 1 or the image storage server 2, medical image V through the network 9. The image interpretation report display mean 30 generates and displays an electronic image interpretation report. The lesion region input means 10 inputs the position of the lesion region in the medical image. The link character insertion means 20 inserts, into the image interpretation report, a link character linked by a hyperlink to the input position of the lesion region in such a manner that the link character is distinguishably displayed. The link character selection means 40 selects the displayed link character in the displayed image interpretation report. The index display means 60 displays, based on the selection by the link character selection means 40, an index representing the position of the lesion region in the medical image.

The lesion region input means 10 mainly includes the input unit 303 for inputting a lesion region. The lesion region input means 10 inputs, into the link character insertion means 20, information representing the position of a lesion region and information representing medical image V including the position of the lesion region, as position information. The position of the lesion region is appropriately input, as a point, a circle, a rectangle or the like, to the medical image V by an operation by an operator at the input unit 303.

The link character insertion means 20 mainly includes the CPU 307. The link character insertion means 20 links, by a hyperlink, the position of the lesion region input by the lesion region input means 10 and a predetermined character string composed of a character string, a sign, a number or the like that has been prepared in advance to each other. Further, the link character insertion means 20 inserts, into the image interpretation report prepared by the image interpretation report display means 30, the predetermined character string as a link character in such a manner that the link character is distinguishably displayed.

The image interpretation report display means 30 mainly includes the input unit 303 for generating an image interpretation report, the CPU 307 for generating the image interpretation report, and the display unit 301 for displaying the image interpretation report. The image interpretation report display means 30 generates the image interpretation report that reflects an input at the input unit 303 based on an operation by an operator, and displays the generated image interpretation report on the display unit 301.

The link character selection means 40 mainly includes the input unit 303. The link character selection means 40 inputs, based on the selection of a link character by the operator at the input unit 303, the selected link character into the index display means 60 and the medical image display means 50.

The medical image display means 50 mainly includes the communication interface 311 for obtaining medical image V from the network 9, and the display unit 301 for displaying the obtained medical image V. The medical image display means 50 displays, on the display unit 301. the medical image obtained through the network.

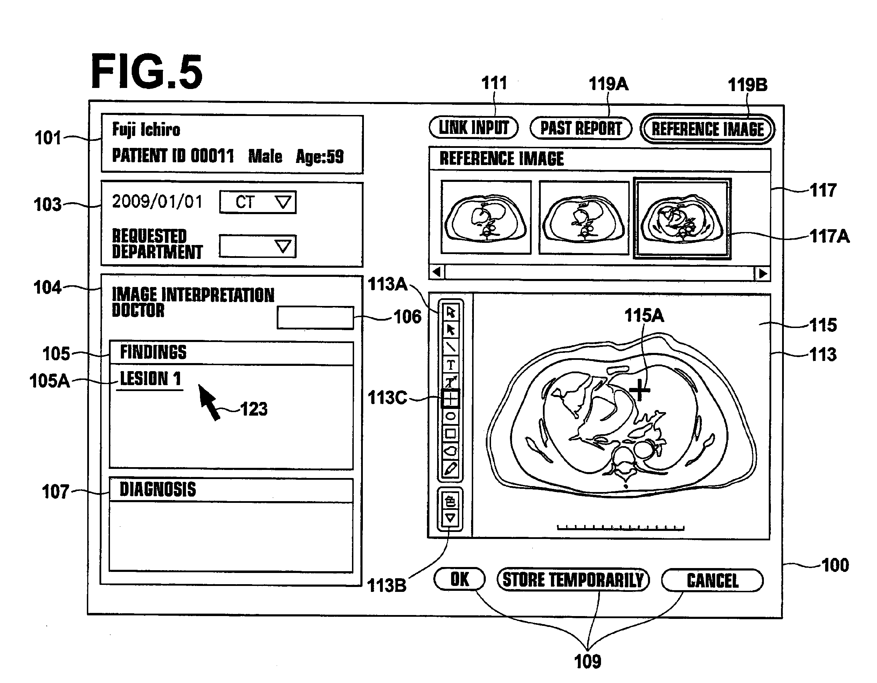

The index display means 60 mainly includes the display unit 301 for displaying an index. The index display means 60 displays an index at the position of a lesion in medical image V that is linked to the selected link character. At this time, the medical image V including the position of the lesion corresponding to the selected link character is displayed on the display unit 301 by the medical image display means 50. The index may be a cross mark, such as an index at position 115A illustrated in Figure 5. Alternatively, a known index, such as a point, a circle, a rectangle, an arrow and a closed curve, which can represent a position may be used.

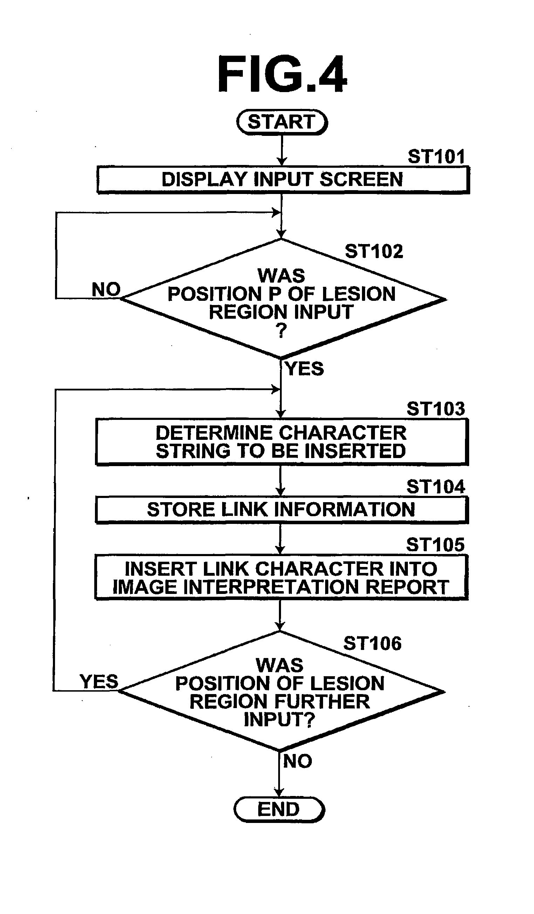

With reference to Figures 4 and 5, an operation for inserting a link character into an image interpretation report according to the present embodiment will be described.

Figure 5 is a conceptual diagram of an image interpretation report and a medical image displayed by the image interpretation report generation function according to the first embodiment. Figure 5 illustrates an image interpretation report generation screen 100, as an example of the image interpretation report generation screen.

First, the composition of the image interpretation report generation screen 100, illustrated in Figure 5, will be described. The image interpretation report generation screen 100, illustrated in Figure 5, includes a patient information area 101 for describing information about a patient. The patient information area 101 displays, for example, a patient's name, a patient's ID, sex, age, and the like, which are obtainable from head information of DICOM. Further, the date of imaging, a modality, a requested department, and the like are input to a medical image information area 103 for showing information about medical image V by the input unit 103, such as the mouse and the keyboard, or based on the header information of DICOM, and displayed. Further, an image interpretation report area 104 includes a box 106 of the name of a doctor who performs image reading, a box 105 of findings, which are an image interpretation report, and a box 107 of diagnosis. A doctor or the like, as an operator, can input data into the box 106 of the name of a doctor who reads the image, the box 105 of findings, and the box 107 of diagnosis, and edit the data by using a mouse, a keyboard, or the like.

Further, the image interpretation report generation screen 100 may appropriately include an image interpretation edit button 109 for storing or cancelling an edited image interpretation report, or the like, and various other function buttons, if necessary. In Figure 5, a temporary storage button, an OK button, a cancel button, and the like are illustrated as examples.

In the image interpretation report generation screen 100, buttons, such as a past report selection button 119A and a reference image selection button 119B, which are necessary to refer to information, are appropriately prepared. Further, the image interpretation report generation screen 100 includes a link input selection button 111 that can make an input of link possible in the embodiments of the present invention. The link input selection button 111 may be provided in various known manners as long as the purpose of the button is achievable.

When the past report selection button 119A is selected, a past report is selectably displayed in a reference information area 117. When the reference image selection button 119B is selected, a thumbnail of medical image V is selectably displayed in the reference information area 117, as illustrated in Figure 5.

Further, the image interpretation report generation screen 100 includes a detail image display area 113. Further, reference information, such as a thumbnail of medical image V and a past report, is appropriately displayed in the reference information area 117. Figure 5 illustrates a state in which a thumbnail 117A is selected from plural thumbnails representing medical image V in the reference information area 117, and a medical image 115 corresponding to the thumbnail 117A is displayed in the detail image display area 113. Here, the plural thumbnails displayed in the reference information area 117 may be thumbnails of medical images representing a lesion included in a series of medical images V. Alternatively, the plural thumbnails may be thumbnails of medical images representing lesions included in plural series of medical images V.

The detail image display area 113 appropriately includes edit buttons 113A and 113B for processing and editing a medical image displayed in the detail image display area 113.

Next, with reference to Figure 4, the flow of inputting a link character 105A to the box 105 of findings, which is an image interpretation report, will be described. Figure 4 is a flowchart illustrating the operation by the image interpretation report generation apparatus according to the first embodiment.

First, the link input button 111 is selected by using the input unit 303, and a link input mode is turned on (ST101).

Next, a cross pointer 113C is selected from the edit button 113A by using the input unit 303 to input the position of a lesion. Then, the cross pointer is displayed. When the cross pointer is positioned at the lesion, and the mouse is clicked at the position, the position 115A of the lesion is input (ST102 is Y) . In this manner, the lesion region input means 10 in the image interpretation report generation apparatus of the present embodiment can manually input the position of a lesion region. Further, selection of the position 115A of the lesion is not limited to the selection by the edit button 113A illustrated in Figure 5, and the position of the lesion region may be selected or displayed by using various marks (indices), such as a point, a circle, a rectangle, a closed curve, and an arrow, as long as the marks are a figure, a line, and a point that can provide marking. Further, the color of the mark may be selected by using a tool, such as a color selection button 113B.

Next, when the position 115A of the lesion region is input, information representing the storage location of the medical image 115 including the position 115A of the lesion, and information including position information, such as the coordinate of the lesion region in the medical image 115, representing the position of the lesion region are obtained by the link character insertion means 20. Further, the position information may include information, such as an image ID, obtained from the header information of DICOM for example. The position information may include image information, such as the luminance value of the image at the position 115A of the lesion. For example, when the position of the lesion region is a point, a coordinate value in the medical image 115 may be obtained as the position information. When the position of the lesion region is a circle, the coordinate value of the center of the circle in the medical image 115 and the radius of the circle may be obtained as the position information. When the position of the lesion region is a figure that is a shape enclosed by straight lines or a curve, information representing the figure, such as the end points of the straight lines in the medical image 115 and a function representing the curve, may be appropriately obtained as the position information.

The link character insertion means 20 selects, from predetermined character strings, a character string that has not been used in the box 105 of findings, and determines the character string as the link character 105A to be inserted (ST103).

The predetermined character strings may be any one of character strings, signs, and numbers. As the predetermined character strings, (1) a fixed pattern character string, such as LESION 1 and LESION 2, (2) when medical image V is composed of a series of slice images, a number that is a combination of the series number of the medical image V and the slice number of a slice image including the position of the lesion together, (3) a pixel value at the position of the lesion, (4) the anatomical name of the region at the position of the lesion, (5) the coordinate value of the position of the lesion, or (6) a combination of these elements may be used.

When the fixed pattern character string, such as LESION 1 and LESION 2, is used as the predetermined character string, it is possible to perform a linking operation only by selecting the position of the lesion region without inputting a link character at the input unit 303. Therefore, it is possible to more easily generate an image interpretation report including a hyperlink. Further, since it is possible to insert the link character only by manually selecting the position of the lesion region, it is possible to more easily generate an image interpretation report including a hyperlink.

Further, when a number that is a combination of the series number of the medical image 115 and the slice number of the slice image including the position of the lesion is used as the predetermined character string, the link character is generated by obtaining necessary information from the header information of the medical image 115. In this case, a linking operation is possible without inputting a link character at the input unit 303, and it is possible to clearly recognize a medical image to which the character string is linked. Therefore, it is possible to more easily generate an image interpretation report including a hyperlink.

Further, when a pixel value or a coordinate value at the position of the lesion is used as the predetermined character string, the link character is generated by obtaining a corresponding pixel value or a corresponding coordinate value at the position of the lesion region in the medical image 115. In this case, a linking operation is possible without inputting the link character from the input unit 303, and it is possible to estimate the condition and the position of the lesion region from the link character. Therefore, it is possible to more easily generate an image interpretation report including a hyperlink.

Further, when a region name representing an anatomical region is used as the predetermined character string, it is possible to extract and insert the link character, as described below. First, an organ to which the input position of the lesion region belongs is extracted by CAD (CAD; Computer Aided Diagnosis). As examples of organ extraction, the following techniques are applicable. Specifically, Japanese Unexamined Patent Publication No. 2001-137230, and Japanese Unexamined Patent Publication No. 2008-253293 are applicable to lung fields. Japanese Unexamined Patent Publication No. 2001-283191, and Japanese Unexamined Patent Publication No. 2002-345807 are applicable to extraction of a liver. Japanese Unexamined Patent Publication No. 2008-43564 is applicable to bones. Japanese Unexamined Patent Publication No. 2004-141612 is applicable to a heart. Further, other organ recognition techniques are applicable as long as the techniques can automatically extract an organ to which the selected position of the lesion belongs. For example, the aforementioned organ extraction is performed on a medical image including the position of a lesion, and if an extracted organ includes the position of the lesion, the organ may be determined as the organ to which the position of the lesion belongs. A region name representing this organ, and which represents an anatomical region, may be inserted as the link character. In this case, a linking operation is possible without inputting a link character at the input unit 303, and it is possible to estimate the condition or the position of the lesion region from the link character. Therefore, it is possible to more easily generate an image interpretation report including a hyperlink.

Further, a region name representing each organ or a region of each organ may be used as the region name. For example, as for a lung, a left lung and a right lung may be used as the region names, or smaller classified regions, namely, superior lobe of right lung, middle lobe of right lung, inferior lobe of right lung, superior lobe of left lung, and inferior lobe of left lung may be used as the region names. Alternatively, regions classified into bronchopulmonary segments, such as superior lingular bronchopulmonary segment (S4), may be used as the region names. Further, abbreviations of these names may be used as the region names.

Further, in a modified example of using, as the predetermined character string, a region name representing an anatomical region, a lesion name storage means 80 for storing a link character candidate representing a lesion may be further provided. When the lesion name storage means 80 is provided, it is possible to register the name of a lesion, the name of a region, or the abbreviation thereof, in advance, in the lesion name storage means 80. Further, the stored lesion names may be selectively used as the predetermined character string.

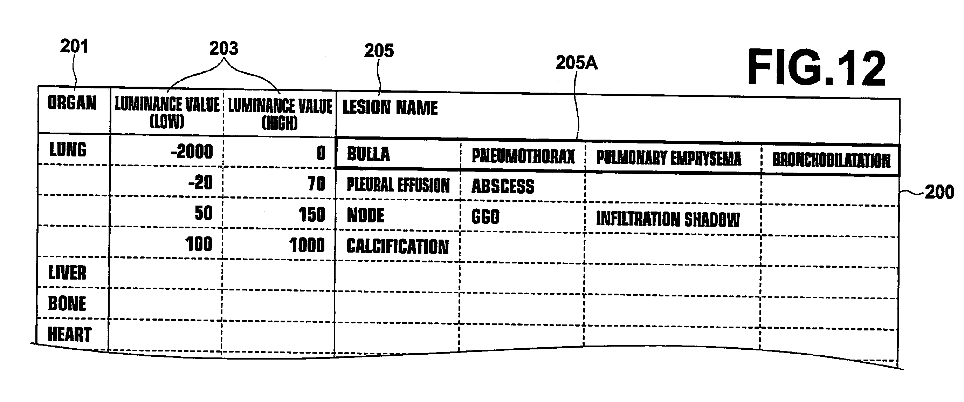

The lesion name storage means 80 is mainly composed of a hard disk 305. The lesion name storage means 80 is a database of lesion names in which at least two lesion names are stored. Plural lesions or regions are registered in advance. The lesion name may be registered by an input at the input unit 303. Alternatively, lesion names that have been prepared in advance may be copied through a network, a recording medium or the like, and generated. Further, an anatomical region name, such as an organ, may be registered in the lesion name storage means 80. Here, the lesion name may be an abbreviation of the name or a sign representing the lesion. Specifically, a correspondence table of the positions of lesion regions and link character candidates, as illustrated in Figure 12, is prepared in the lesion name storage means 80. The correspondence table shows correspondence among organ names 201, characteristic information 203 about the positions of lesion regions, and candidates 205 of link characters representing lesions. In Figure 12, the luminance values 203 of a CT image, as the characteristic information 203 about the positions of lesion regions, are corresponded.

When the position of a lesion region is input, an organ to which the input position of the lesion region belongs is extracted by using the aforementioned example of organ extraction by CAD. Next, the luminance value of an image at the input position of the lesion region is obtained as position information.

As illustrated in Figure 12, when the input position of the lesion region belongs to the lung in the organ name 201, and for example, when the luminance value at the position of the lesion region, which has been input with respect to the characteristic information 203 at the position of the lesion, is -50, which is included in the range of from -2000 to 0, four character strings of the link character candidates 205 are determined, as corresponding link character candidates 205A. The four character strings are BULLA, PNEUMOTHORAX, PULMONARY EMPHYSEMA, and BRONCHODILATATION. When the luminance value is 100, the luminance value of 100 is included in the range of from 50 to 100, and also in the range of from 100 to 1000. Therefore, NODE, GGO, INFILTRATION SHADOW, and CALCIFICATION are determined as link character candidates.

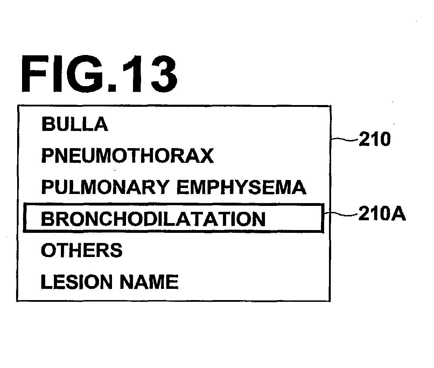

As illustrated in Figure 13, when a user selects the position of a lesion region, the link character candidates 20A are displayed as a link character candidate list 210 in the image interpretation report generation screen 100. When a user selects a link character candidate 210A from the link character candidate list 210, the selected link character candidate 210A is inserted into the image interpretation report, as a link character. In Figure 13, when a character string that is not the link character candidate is input, OTHERS may be selected, and a link character may be input at the input unit. The link character candidate list may be displayed in pull-down menu form. Alternatively, various methods that can select a region name are adoptable. In this case, a linking operation is possible only by selecting a link character at the input unit 303, and it is possible to estimate the condition or the position of the lesion region from the link character. Therefore, it is possible to more easily generate an image interpretation report including a hyperlink.

Further, an average or a variance of luminance values in the vicinity of the position of the lesion region, or the pattern of luminance values may be obtained, as information obtained from the position of the lesion. Further, corresponding link character candidates may be selected by using, as characteristic information 203 about the position of lesion region, characteristic information based on an average or a variance of luminance values in the vicinity of the position of the lesion region, or the pattern of luminance values.

Next, the link character insertion means 20 stores link information about the position 115A of the lesion (ST104). For example, the link information may be composed of (1) a link ID, (2) the name of a series, a slice name, and the position of the lesion region represented by a coordinate on the slice, and (3) a link character. The link ID, the name of a series, the slice name, the position of the lesion region represented by a coordinate on the slice are obtainable from position information input from the lesion region input means 10.

A link character 105A is linked, by a hyperlink, to an image including the position 115A of the lesion region obtained from the lesion region input means 10, and inserted to the box 105 of findings (ST105). At this time, the link character 105A is inserted in such a manner that the state of linking by a hyperlink is identifiable. The link character 105A is distinguished from characters other than the link character 105A by means of coloring the link character or the background of the link character, an underline, blinking, bold letters, a letter type, the size of the link character, and a frame. As for an insertion position, it is desirable that the link character is inserted to a position at which a cursor is located in the image interpretation report. Alternatively, the link character may be inserted after the last sentence in the box 105 of finding in the image interpretation report. The link character may be appropriately inserted to a desirable position.

Further, when the position of the lesion region is input in a manner similar to ST102, the steps from ST102 to ST105 are repeated (ST106 is Y). In other words, the lesion region input means 10 of the present embodiment can input the positions of plural lesion regions, and the link character insertion means 20 can insert plural link characters corresponding to the input positions of the lesion regions, respectively. When the positions of plural lesion regions are selected in the medical image V, corresponding link characters are inserted the same number of times as the number of selected positions.

When the position of a lesion region is not input, link character insertion processing ends (ST106 is N).

Further, when the input unit 303 has selected the inserted link character in the image interpretation report generation area 105, for example, when the link character 105A has been selected by a selection tool 123 or the like illustrated in Figure 5, the medical image display means 50 obtains position information about a medical image 115 corresponding to the link character 105A from the image interpretation report display means 30. Further, the medical image display means 50 displays the medical image 115 in the detail image display area 113. Further, the index display means 60 obtains link information from the link character insertion means 20, and displays the position 115A of the lesion region corresponding to the link character 105A on the medical image 115 in the detail image display area 113. It is desirable that the index is displayed in the same style as the input of the position of the lesion region. Specifically, if the position of a lesion region is input with a cross pointer, it is desirable that the index is represented also by a cross pointer. If the position of a lesion region is input with a closed curve, it is desirable that the index is represented also by a closed curve. Here, when the position of a lesion region is represented as an area, the index may be displayed as a representative point in the area. Further, the display style of the index may be selected from an arrow, an area, various pointers indicating a representative point in the lesion region, and the like.

An operator can edit an inserted link character and sentences around the link character by an operation of a keyboard or the like after insertion of the link character. Further, a different link character may be inserted. Further, an input of link and an operation of writing an image interpretation report may be performed alternately.

Meanwhile, in conventional techniques, it was possible to check a medical image including a lesion region only after generating a hyperlink in a generated image interpretation report by drag-and-drop. However, in the present invention, it is possible to generate link characters before a specific content is written in the image interpretation report by selecting, one after another, the positions of lesion regions in medical image V. Further, when a link character that has been already inserted is selected even while an operator is writing the content of the image interpretation report, it is possible to display the position of a corresponding lesion region. Therefore, it is possible to easily check the position of a lesion region during generation of the image interpretation report. Further, it is possible to more easily generate an image interpretation report including a hyperlink.

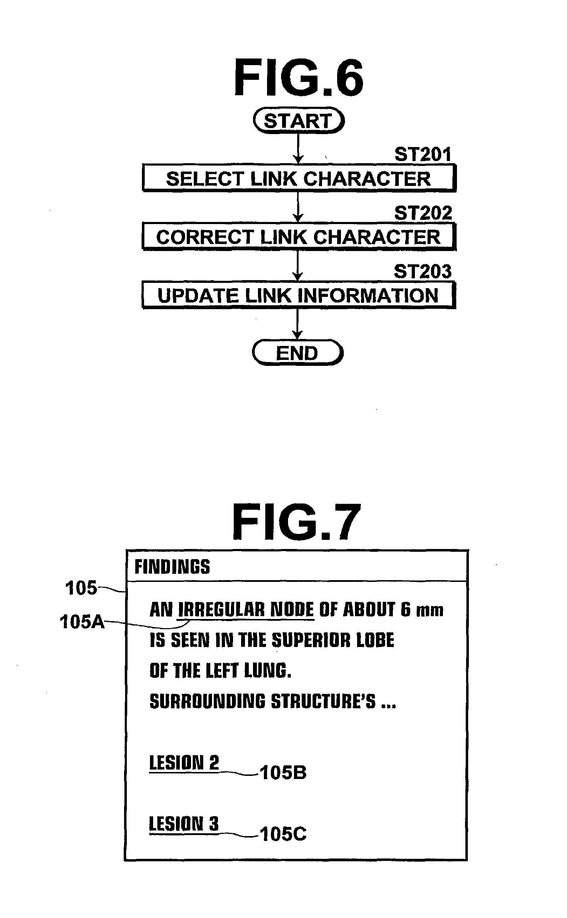

With reference to Figures 6 and 7, an operation of changing a link character inserted in the image interpretation report according to the present embodiment will be described.

Figure 6 is a flowchart for explaining the flow of changing a link character in the first embodiment.

Figure 7 is a conceptual diagram of an image interpretation report generated by changing a part of the link character in the first embodiment.

Figure 7 illustrates an example of the box 105 of findings in the image interpretation report generation screen 100. Figure 7 illustrates a state in which three link characters, namely, LESION 1, LESION 2 (105B), and LESION 3 (105C) have been inserted, and LESION 1 is edited to IRREGULAR NODE (105A), which is a new link character.

First, in the box 105 of findings in which the link characters have been inserted, the input unit 303 selects a link character (ST201). Next, the link character is changed to an arbitrary character string, or sign, or number by an operation of a mouse or a keyboard. Specifically, for example, a selection tool, such as an arrow 123, is positioned at the link character, and the mouse is right clicked. Then, a link character change option is displayed in pull-down menu form. The link character is changed by selecting the link character change option (ST202). The link character may be changed by using other appropriate methods as long as the methods can change only the character strings of the link characters while the state of the hyperlink is maintained.

Next, the link information is updated by linking, to the position of the lesion region, the link character after change, instead of the link character before change (ST203).

When the position of the lesion region is selected, as a point, by the cross pointer or the like, as described above, a link character linked, by a hyperlink, to the position of a lesion region is inserted to the image interpretation report only by a click of the input unit 303. Therefore, unlike conventional techniques, it is not necessary to repeat drag-and-drop operations. Therefore, it is possible to easily link the position of a lesion region. Further, the position of the lesion region is selected as a point or an area, and the selected position is displayed in the medical image V. Therefore, it is possible to immediately recognize a part of the medical image V that is referred to in the image interpretation report. Hence, it is possible to more easily understand a medical image corresponding to the linked character string.

Further, when the positions of plural lesion regions are indicated in a medical image, the positions 115D, 115E, and 115F of the plural lesion regions may be displayed in such a manner that when the link character 105D is selected, the position 115D is displayed but the positions 115E and 115F are not displayed.

In the technique disclosed in Patent Document 1, a character string is linked to an image. Therefore, when a slice image includes plural link target regions, the corresponding medical image is the same image, irrespective of selection of any character string from the linked plural character strings. Hence, there has been a problem that it is difficult to recognize which position in the medical image each character string indicates. However, in the present embodiment, an index representing the position of a lesion region is displayed in the medical image. Therefore, it is possible to clearly recognize about which position of a lesion region each link character 105D describes. Hence, it is possible to more easily understand a medical image corresponding to the linked character string.

Further, when a slice image includes plural link target regions, a complicated method as disclosed in Patent Document 1 is not used. In the technique disclosed in Patent Document 1, plural linked target regions are cut out from a slice image, and new plural reference images are generated for respective target regions. Further, a linking operation is performed by drag-and-drop of each of the generated reference images to corresponding character strings, respectively. Unlike Patent Document 1, it is possible to generate an image interpretation report that can accurately recognize the position of a lesion region corresponding to a link character only by selecting and inputting a corresponding position at the input unit 303. Therefore, it is possible to more easily generate a medical image including a hyperlink.

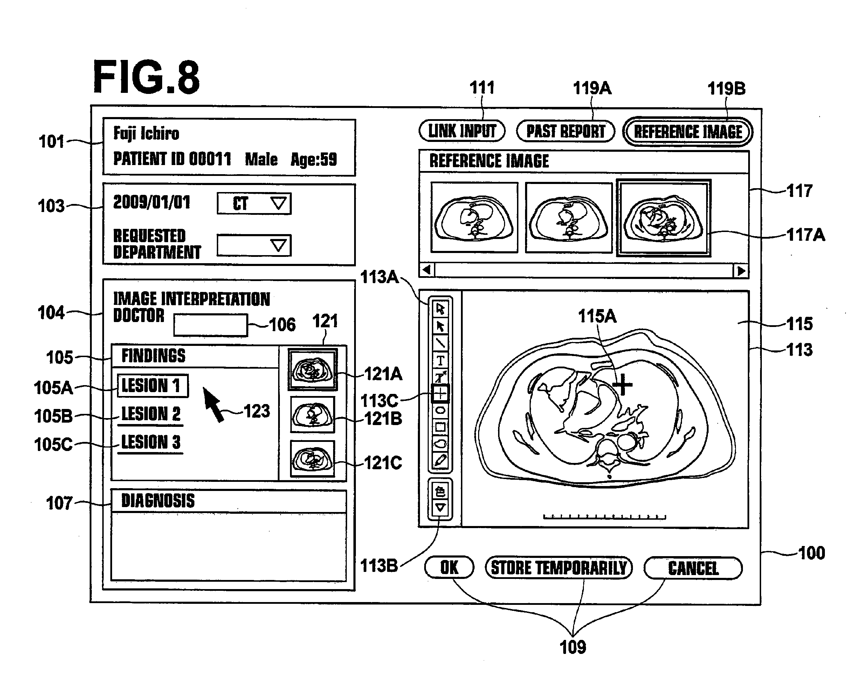

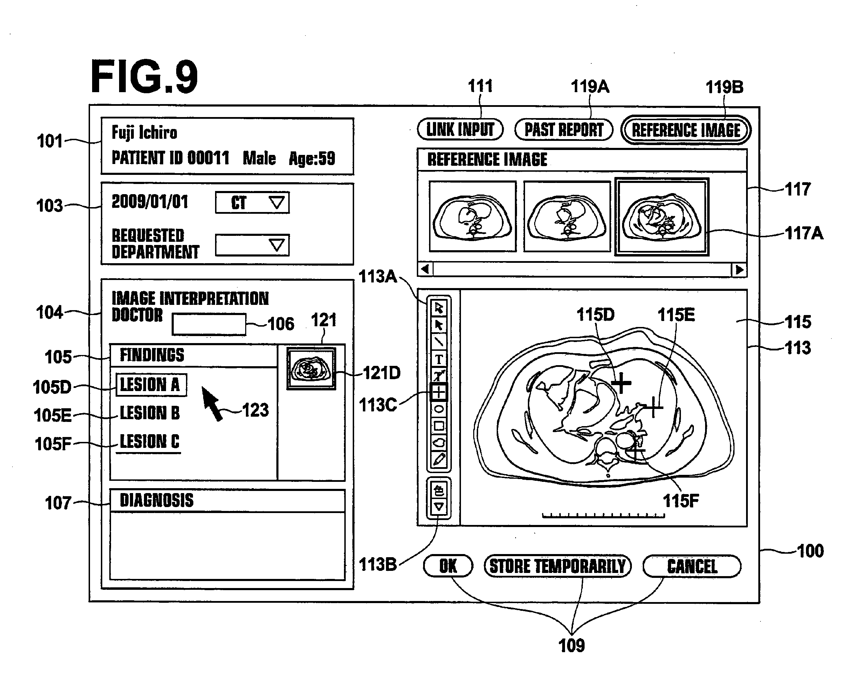

Figure 8 is a conceptual diagram of an image interpretation report and a medical image displayed by an image interpretation report generation function of the second embodiment. In Figures 8 and 9, the composition of elements to which the same numbers as those in Figure 5 are assigned is same as Figure 5. Therefore, explanation of the elements will be omitted.

As illustrated in Figure 8, in the image interpretation report generation screen, the image interpretation report display means 30 includes an attachment image display area 121 in an image interpretation report area 114, and displays, as attachment images, thumbnails 121A, 121B, and 121C together with the image interpretation report. The thumbnails 121A, 121B, and 121C are reduced medical images including the positions of lesion regions corresponding to the link characters. The thumbnail 121A of the medical image is a reduced medical image including the position 115A of the lesion region. The thumbnail 121B is a reduced medical image including the position 115B of the lesion region. The thumbnail 121C of the medical image is a reduced medical image including the position 115C of the lesion region.

If a thumbnail (reduced image) of a medical image including the position of a lesion region corresponding to a link character is displayed together with an image interpretation report as described above, it is possible to more easily understand the medical image corresponding to the linked character string when the image interpretation report is read later.

Further, when thumbnails of medical images including the positions of the lesion regions, and which are attachment images, are displayed in the attachment image display area 121 of the medical image in the order of description in the report, it is possible to easily recognize about which image each link character describes. Therefore, it is possible to more easily understand the medical image corresponding to the linked character string.

Figure 9 illustrates another modified example. The following modified example is applicable to other embodiments without changing the essential features thereof. Figure 9 is a conceptual diagram of an image interpretation report and a medical image displayed by an image interpretation report generation function in a modified example of the second embodiment.

As another modified example of a case of indicating the positions of plural lesion regions in a medical image, there is a method of displaying the positions 115D, 115E and 115F of the plural lesion regions, simultaneously. In this case, when the link character 105D is selected, the selected link character 105D and the position 115D of the lesion region are displayed distinguishably from the other link characters 105E and 105F and the positions 115E and 115F of the lesion regions, respectively. Accordingly, it is possible to clearly recognize about which position of a lesion region the link character 105D is written. Further, it is possible to recognize the number of the positions of lesion regions indicated in the image. Therefore, it is possible to more easily understand a medical image corresponding to the linked character string. In Figure 9, as an example of distinguishably displaying the positions of plural lesion regions present in a medical image, the link character 105D is selected, and the selected link character 105D is framed. Consequently, the link character 105D is displayed distinguishably from the link characters 105E and 105F. Further, a cross pointer at the position 115D of the lesion region corresponding to the selected link character 105D is made thicker than cross pointers at positions 115E and 115F representing the positions of the other lesion regions. Accordingly, the positions of the lesion regions are distinguishably displayed.

Further, when the selected link character and an index representing the position of a corresponding lesion region are displayed in an emphasized manner by using various distinguishingly-displayable methods, it is possible to more clearly indicate to which link character the position of a displayed lesion region corresponds. The various distinguishingly-displayable methods can distinguishably display by means of blinking of a mark, a character or both of them, coloring of the link character or the background of the link character, an underline, blinking, bold letters, letter types, the size of the link character, a frame, and the like. Hence, it is possible to more easily understand the medical image corresponding to the linked character string.

Further, when plural link characters and the positions of plural lesion regions are present, a link character and the position of a lesion region corresponding to each other may be displayed in the same color. When they are distinguishably displayed in such a manner, it is possible to clearly recognize about which position of the lesion region each link character describes. Further, it is possible to recognize the positions of lesion regions represented by all of the link characters, respectively, at the same time. Therefore, it is possible to more easily understand the medical image corresponding to the linked character string.

Next, a third embodiment will be described. In the third embodiment, the position of a lesion region is automatically input by CAD (CAD; Computer Aided Diagnosis).

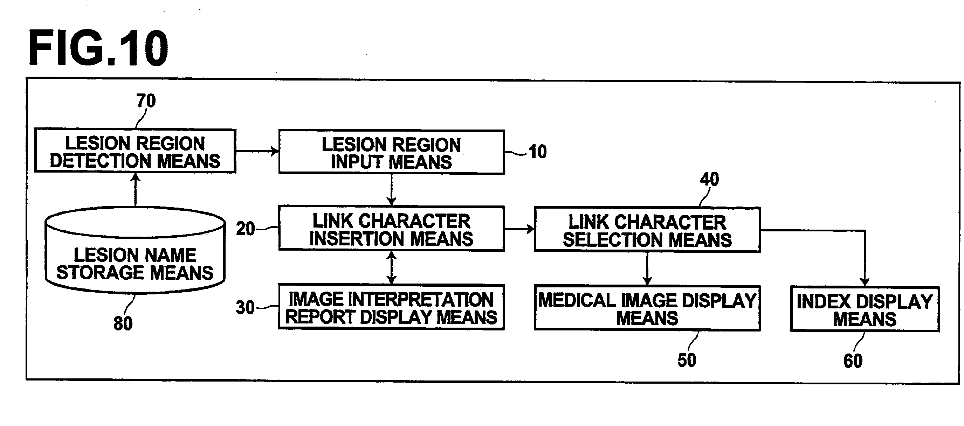

Figure 10 is a functional block diagram illustrating an image interpretation report generation function of the third embodiment. The apparatus further includes a lesion name storage means 80 and a lesion region detection means 70. The lesion name storage means 80 stores plural lesion names representing lesions, and the lesion region detection means 70 automatically detects the position of a lesion region in a medical image. Further, the lesion region input means 10 inputs the position of the lesion region that has been automatically detected by the lesion region detection means 70. Further, the link character insertion means 30 selects, from the lesion name storage means 80, a lesion name representing a lesion located at the position of the lesion region detected by the lesion region detection means 70, and inserts the lesion name. Other features are similar to those of the first embodiment. Therefore, explanation will be omitted with respect to a part to which the same names as those of the first embodiment are assigned.

The lesion name storage means 80 mainly includes a hard disk 305, and the lesion name storage means 80 is a lesion name database in which at least two lesion names are stored. Plural lesions or regions are registered in advance. The lesion names may be registered by an input from the input unit 303. Alternatively, lesion names that have been prepared in advance may be copied through a network, a recording medium or the like, and generated. Further, an anatomical region name, such as an organ, may be registered in the lesion name storage means 80. Here, the lesion name may be an abbreviation of the lesion name or a sign representing the lesion.

The lesion region detection means 70 automatically extracts a lesion from medical image V by using a known means for detecting a lesion, such as a tumor. The lesion region detection means 70 detects the position of a lesion region, and inputs the position of the lesion region into the lesion region input means 10. Further, the link character insertion means 30 selects, from the lesion name storage means 80, a lesion name representing the lesion region detected by the lesion region detection means, and inserts the lesion name as a link character. Here, when link characters represent the same lesion name, it is desirable to insert specific disease names as a subordinate concept for better recognition.

The lesion region detection means 70 performs CAD (CAD; Computer Aided Diagnosis), in which an abnormal shadow candidate is automatically detected by computer processing, and automatically detects a lesion region. In this case, the position of the abnormal shadow may be a region. Alternatively, the position of the abnormal shadow may be a point, such as the center of gravity of the abnormal shadow, which represents a representative position of the abnormal shadow region. Further, automatic detection of lesion regions may be performed on all of medical images, or a part of the medical images.

As techniques for detection of a lesion region, the following techniques may be adopted. Specifically, techniques for detecting a lung cancer disclosed in Japanese Unexamined Patent Publication No. 2003-225231, Japanese Unexamined Patent Publication No. 2003-271924, and K. Kubota et al., "Evaluation of Computer-Aided Diagnosis system for Lung Cancer based on Helical CT images", the Institute of Electronics, Information and Communication Engineers (IEICE), IEICE Technical Report, pp. 41-46, 2001 are applicable. Further, consolidation disclosed in S. Kido et al., "Intelligent CAD for diffuse lung diseases", Grant-in-Aid for Scientific Research, granted by the Ministry of Education, Culture, Sports, Science and Technology (MEXT), Study in Specific Field "Intellectual Diagnosis Aid of Multi-Dimensional Medical Image", Proceedings of 4th Symposium, pp. 45-54, 2007 is applicable. Further, Ground-Glass Opacity(GGO) and Crazy-Paving are applicable. Further, detection techniques of diffuse lung disease, such as honeycomb-shaped shadow, pulmonary emphysema shadow and particle-shaped shadow, are applicable. Further, a technique for detecting a liver cancer disclosed in Y. Wakida et al., "Liver Cancer Detection based on a Temporal Density Feature from Abdominal Dynamic X-ray CT Images", Proceedings of Japan Society of Computer-Aided Diagnosis of Medical Images, Vol. 10, No. 1, 2007 is applicable. Further, a technique for detecting hepatocellular carcinoma, hepatic cyst, hepatic hemangioma, and bleeding in a liver region disclosed in H. Fujita et al., "Intelligent Computer-aided Diagnosis Based on Normal Structure Recognition of Human Body", Grant-in-Aid for Scientific Research, granted by the Ministry of Education, Culture, Sports, Science and Technology (MEXT), Study in Specific Field "Intellectual Diagnosis Aid of Multi-Dimensional Medical Image", Proceedings of 4th Symposium, pp. 55-60, 2007 is applicable.

Further, a technique for detecting an abnormality in a blood vessel, as disclosed in Japanese Unexamined Patent Publication No. 2004-329929, a technique for detecting an abnormal shadow candidate, as disclosed in Japanese Unexamined Patent Publication No. 10 (1998)-97624, which was filed by the applicant of this application, and a technique for detecting a calcified region, as disclosed in Japanese Unexamined Patent Publication No. 8(1996)-215183, may be used. When a lesion region has been detected by using the aforementioned lesion detection techniques, lesion region detection information is input to the lesion region detection means 10. The lesion region detection information specifies the position of the lesion region and a detection technique used to detect the lesion.

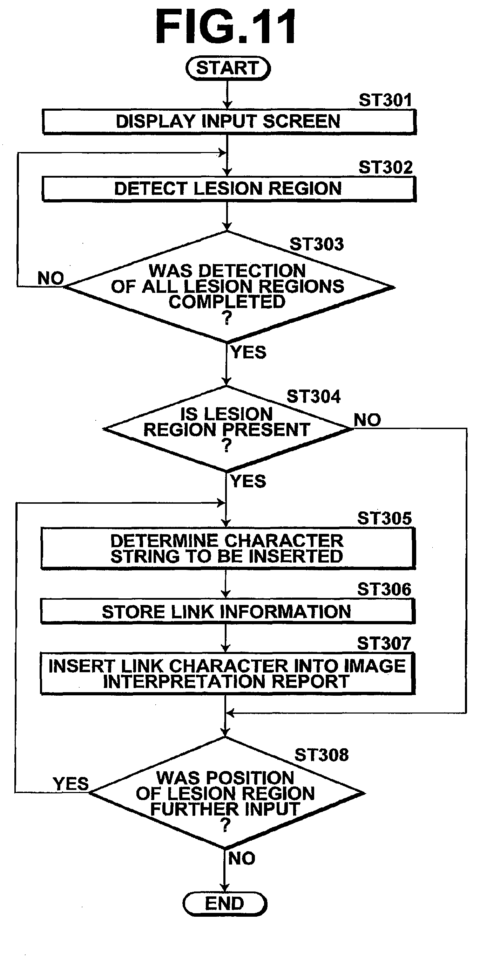

Figure 11 is a flowchart for explaining a flow of link character insertion processing in the third embodiment. With reference to Figure 11, the flow of link character insertion processing in the third embodiment will be described. Here, steps ST305 to ST308 are the same as steps ST103 to ST106, respectively.

First, in a manner similar to ST101, the image interpretation report is set in a condition in which an input of link is possible (ST301). For example, as illustrated in Figure 5, the link input button 111 may be selected by the input unit 303. Further, an automatic lesion region insertion button, or the like may be provided, and a button for making input of a link character possible, as described in the third embodiment, or the like may be provided. The buttons may be turned on to display an input screen.

Next, when the link input mode is turned on, the lesion region detection means 70 automatically detects a lesion, such as an abnormal shadow (ST302). When the lesion region detection means 70 has a function for detecting plural lesions, automatic lesion region detection is repeated until detection of lesion regions is completed by all of the lesion region detection functions (ST303 is N).

When all of lesion region detection is completed (ST303 is Y), and the position 115G of a lesion region is detected, lesion region detection information that specifies the position 115G of the detected lesion region and the method used to detect the position 115G of the lesion lesion is input to the lesion region input means 10. In a manner similar to ST103 illustrated in Figure 4, when the position 115G of the lesion region is input, the lesion region input means 10 obtains information, such as an address representing the storage location of the medical image 115 or an image ID, and a coordinate representing the position 115G of the lesion region. Further, the lesion region input means 10 inputs the lesion region detection information that specifies the method used to detect the position 115G of the lesion lesion to the link character insertion means 30.

The link character insertion means 30 selects, from predetermined character strings, a character string that has not been used in the box 105 of findings, and determines the character string as the link character 105G to be inserted (ST305).

The predetermined character strings are stored, in advance, in the storage means 80 in such a manner that character strings to be inserted are correlated to methods used to detect the positions of lesion regions, and the character string to be inserted is determined based on the input lesion region detection information. Further, it is desirable that the character string to be inserted is a character string representing the detected lesion region.

When the technique used to detect a lesion detects a lesion that is specific to an organ, it is desirable that a keyword representing a lesion name of a detection target by the lesion detection technique is used as a character string to be inserted. Specifically, when the technique used to detect the position of a lesion region is a technique for detecting a lung cancer, "LUNG CANCER" is used as a character string to be inserted. When the technique used to detect the position of a lesion region is a technique for detecting a liver cancer, "LIVER CANCER" is used as a character string to be inserted. In this manner, it is desirable that keywords representing expected lesions are correlated to lesion region detection information specifying techniques used to detect lesions so that the keywords are used as character strings to be inserted. Consequently, the automatically inserted link character is a keyword representing a lesion specific to an organ. Therefore, it is possible to easily recognize the position of a lesion and the name of the lesion based on the link character. Further, it is possible to more easily generate an image interpretation report including a hyperlink.

Further, the region name of a lesion region may be obtained by performing, by using CAD, region recognition on medical image V1 including the lesion region detected by the lesion region detection means 70. Further, a character string composed of the lesion name representing the detected lesion and the obtained region name may be used as a link character to be inserted. Consequently, the automatically inserted link character is a keyword representing the organ and the lesion. Therefore, it is possible to easily recognize, based on the link character, the organ in which the lesion is present and the lesion name. Further, it is possible to more easily generate an image interpretation report including a hyperlink. Specifically, with respect to medical image V1 in which a lesion region has been detected, the techniques disclosed in Japanese Unexamined Patent Publication No. 2001-137230, and Japanese Unexamined Patent Publication No. 2008-253293 are adoptable for a lung field. The techniques disclosed in Japanese Unexamined Patent Publication No. 2001-283191, and Japanese Unexamined Patent Publication No. 2002-345807 are adoptable in extraction of a liver. The technique disclosed in Japanese Unexamined Patent Publication No. 2008-43564 is adoptable for bones, and the technique disclosed in Japanese Unexamined Patent Publication No. 2004-141612 is adoptable for a heart. If the position of the detected lesion is included in the extracted organ, it is possible to judge that the organ is a region including the lesion region. Further, other organ recognition techniques are adoptable as long as it is possible to automatically extract an organ to which the position of a detected lesion belongs. As the character string to be inserted, the predetermined character string explained in ST105 may be used.

Next, the link character insertion means 20 stores link information about position 115G (ST306). For example, the link information may be composed of (1) a link ID, (2) a series name, a slice name, and the position of a lesion region represented by a coordinate on a slice, and (3) a link character. The link ID, the series name, the slice name, and the position of a lesion region represented by a coordinate on a slice may be obtained based on the position information input from the lesion region input means 10.

A link character 105G is linked, by a hyperlink, to the position 115G of the lesion region obtained from the lesion region input means 10, and is inserted to the box 105 of findings (ST307) . At this time, in a manner similar to ST105, the link character 105G is inserted in such a manner that the state of linking by a hyperlink is identifiable.

Further, when the position of the lesion region is input at the input unit 303 in a manner similar to ST102, steps of ST305 to ST307 are repeated (ST308 is Y). Specifically, when the positions of plural lesion regions are selected in medical image V, the same number of corresponding link characters as the number of times of selection are inserted.

When no position of a lesion region is input, link character insertion processing ends (ST308 is N). When there is an input by the input unit 303, processing similar to ST103 is performed in ST305, as described above.

If the position of a lesion region to be linked to is automatically input as in the third embodiment, a link character linked, by a hyperlink, to the position of the lesion region has been already inserted in the image interpretation report when generation of the image interpretation report is started. Therefore, it is possible to more easily generate an image interpretation report including a hyperlink. Further, according to the present embodiment, it is possible to provide a list of information about link characters and the positions of corresponding lesion regions for a person who reads the medical image V before he/she reads through the medical image V. Therefore, it is possible to provide an estimated lesion region for the person who reads the image. Hence, it is possible to reduce human errors, such as overlooking a lesion region or forgetting to write information in a report, during interpretation of the image. Accordingly, it is possible to assist generation of a more accurate image interpretation report.

Further, when the character string to be inserted in the image interpretation report is a character string related to a region, it is possible to easily understand in which region the lesion region is present by looking at the automatically inserted link character. Therefore, it is possible to more easily generate an image interpretation report including a hyperlink.

Further, as a modified example of the third embodiment, a lesion region detection means 70' may be provided instead of the lesion region detection means 70. When the input unit 303 selects the position of a lesion region, the lesion region detection means 70' extracts the lesion region including the position of the lesion region.

Specifically, in the flow of link character insertion illustrated in Figure 11, the following process is performed instead of ST302. The process other than ST302 is similar to the third embodiment.

A method disclosed in Japanese Unexamined Patent Publication No. 2007-307358, which was filed by the applicant of the present application, is used. First, machine learning is performed, in advance, on the feature value of each pixel in plural sample images including abnormal regions the outlines of which are known. Accordingly, evaluation function F for evaluating whether each pixel is a pixel representing an outline is obtained with respect to feature values. Next, a position in an abnormal region in the image is selected by using the input unit 303. Further, a sufficiently large identified region including the abnormal region is determined based on the position, and a feature value for each pixel in the determined identified region is extracted. An evaluation value as to whether each pixel represents an outline is obtained, based on the extracted feature value, by using the evaluation function. Further, an optimum outline of the lesion region is obtained based on the obtained evaluation value. The obtained outline of the lesion region is input to the lesion region input means 10.

When the modified example of the third embodiment is used, it is possible to more accurately extract and select a lesion region only by selecting the position of the lesion region as a point. Further, it is possible to generate an image interpretation report showing a more accurate lesion region. Hence, it is possible to generate a higher-quality image interpretation report without complicating an operator's operation.

Further, as another modified example of the present embodiment, an organ region may be automatically input as the position of a lesion region. In this case, an organ region is automatically detected, and lesion region detection information specifying the position of the detected organ region and the detection technique used to detect the organ is input to the lesion region detection means 10. Further, when a link character is inserted, it is desirable that a keyword representing an organ name of a detection target by the technique used to detect the organ region is used as a character string to be inserted. Specifically, when the technique used to detect the position of the organ region is a technique for detecting a lung, "LUNG" is used as a character string to be inserted. It is desirable that keywords representing expected organ names or regions of organs are correlated to lesion region detection information specifying techniques used to detect organ regions so that the keywords are used as character strings to be inserted. Consequently, the automatically inserted link character is a keyword representing the organ. Therefore, it is possible to easily recognize the organ based on the link character. Further, it is possible to more easily generate an image interpretation report including a hyperlink.

Here, with respect to an organ region, an organ including a lesion may be detected in some cases, and an organ without any lesion may be detected in other cases. Even when an organ without any lesion is detected, a comment on the organ without any lesion is still written in an image interpretation report in some cases when the condition of a disease is improved or the like. Therefore, generation of an image interpretation report becomes easy.

As techniques for automatically detecting an organ region, the following techniques are applicable. Japanese Unexamined Patent Publication No. 2001-137230, and Japanese Unexamined Patent Publication No. 2008-253293 are applicable to lung fields. Japanese Unexamined Patent Publication No. 2001-283191, and Japanese Unexamined Patent Publication No. 2002-345807 are applicable to extraction of a liver. Japanese Unexamined Patent Publication No. 2008-43564 is applicable to bones. Japanese Unexamined Patent Publication No. 2004-141612 is applicable to a heart. Further, other techniques are applicable as long as the techniques can automatically detect an organ region.

In the specification of the present application, in the operation of changing the link character that has been inserted in the image interpretation report, as described with reference to Figure 6, editing of the image interpretation report is started in step ST201 of link character selection, and a link character to be edited is selected. At this time, it is desirable to separately provide a mode of editing a link character and a mode of editing a character other than the link character, and to switch the modes to each other between a case of editing a link character and a case of editing a character other than the link character. Further, it is desirable that characters other than the link character in the image interpretation report are locked so that they are not changeable in the mode of changing the link character. In contrast, it is desirable that the link character is locked so that the link character is not changeable in the mode of editing the characters other than the link character in the image interpretation report.

As the method for switching a mode of editing a link character and a mode of editing a character other than the link character, for example, a pull-down menu may be displayed by right clicking a mouse while a selection tool is positioned on the link character in the image interpretation report. Further, the mode of editing a link character and the mode of editing characters other than the link character in the report may be provided to be selectable in the pull-down menu. Alternatively, the mouse may be double clicked while the selection tool is positioned on the link character in the image interpretation report to turn on the mode of editing the link character, and to turn off the mode of editing characters other than the link character at the same time. The mouse may be double clicked while the selection tool is positioned on a character other than the link character to turn on the mode of editing characters other than the link character, and to turn off the mode of editing the link character at the same time. Similarly, the mouse may be right clicked while the selection tool is positioned on the link character in the image interpretation report to turn on the mode of editing the link character, and to turn off the mode of editing characters other than the link character at the same time. The mouse may be right clicked while the selection tool is positioned on a character other than the link character to turn on the mode of editing characters other than the link character, and to turn off the mode of editing the link character at the same time. Further, a button or the like for selecting a mode of editing a link character and a mode of editing characters other than the link character in the report may be provided on the display screen. The method for switching the mode of editing a link character and the mode of editing characters other than the link character is not limited to the aforementioned examples, and various methods for changing modes are applicable.

As described above, the mode of editing the link character and the mode of editing characters other the link character are switched. Therefore, when a new character is inserted before or after a link character, it is possible to clearly judge whether the inserted character is inserted as a link character or as a character other than the link character. Further, it is possible to prevent a user from erroneously editing a link character as a character other than the link character. Further, it is possible to prevent a user from erroneously editing, as a link character, a character other than the link character.

In the embodiments in the specification of the present application, the position of a corresponding lesion region is displayable based on selection of a link character even while the image interpretation report is edited. Therefore, a method for selecting a link character to display the position of a corresponding lesion region and a method for selecting a link character to edit the link character should be different from each other. For example, the link character is selected by left click of a mouse to display the position of a lesion region corresponding to the link character, and the link character is selected by using the methods as described above to edit the link character.

Further, in the steps of ST201 and ST202 in Figure 6, the image interpretation report may be edited by an input of user's voice. For example, a voice input unit, such as a microphone, and a voice recognition means, such as voice recognition software for recognizing the user's voice, may be further provided. Further, a button for switching ON/OFF of image interpretation report voice editing, or the like nay be provided on a display screen in such a manner that ON/OFF is selectable. Further, the input means 303 may turn on the image interpretation report voice edit mode based on selection of "ON" in the image interpretation report voice edit button by the user. Accordingly, when the image interpretation report voice edit mode is ON, all or a part of edit of link characters may be performed, based on voice information obtained by the voice recognition means, by obtaining, at the voice input unit, a voice input by the user. Further, the image interpretation report voice edit mode may be applied to at least one of the link character and characters other than the link character, or to both of the link character and the characters other than the link character.

As the voice edit method, various known methods may be used. For example, when the image interpretation report voice edit mode is ON, if a microphone receives voice saying "Link Character Edit", the first link character of plural link characters described in the image interpretation report is selected, and distinguishably displayed. While the link character is distinguishably displayed, if voice saying "Link Character Candidate" is received, candidate 1 of the link character, candidate 2 of the link character, and candidate 3 of the link character are displayed in a pull-down menu in the vicinity of the distinguishably displayed link character in such a manner that a candidate is selectable therefrom. Here, when voice saying "Select No. 1" is received, the link character may be changed to candidate 1 of the link character. Alternatively, when voice saying "Link Character Input" is received while the link character is distinguishably displayed as described above, the user may change the link character to a word or phrase that is input by voice following the voice saying "Link Character Input" . In the voice edit mode, it is desirable that operations necessary for each edit are performed also by an input of voice. The operations necessary for each edit are selecting one of plural link characters in the image interpretation report when voice saying "Link Character Edit" is received, selecting a link character following the distinguishably displayed link character or a link character before the distinguishably displayed link character in the order of arrangement from the beginning of the image interpretation report when voice saying "Next" or "Before" is received, and the like.

As described above, when it is possible to edit a link character in the image interpretation report based on an input of user's voice, it is possible to generate the image interpretation report also by editing by voice. Therefore, the operation characteristic is improved, and it is possible to easily generate the image interpretation report.

## Claims

1. An image interpretation report generation apparatus comprising:
a medical image display means (50) adapted to obtain and display a medical image;
an image interpretation report display means (30) adapted to generate and display an electronic image interpretation report of the medical image; and
a link character selection means (40) that selects a distinguishably displayed link character in the image int.erpretation report;
**characterized by**
a lesion region input means (10) adapted to manually input the position of a lesion region in the medical image;
a link character insertion means (20) adapted to insert, into the image interpretation report, the link character linked by a hyperlink to the medical image including the input position of the lesion region in such a manner that the link character is distinguishably displayed; and
an index display means (60) that displays, based on the selection by the link character selection means, an index representing the position of the lesion region in the medical image.

2. An image interpretation report generation apparatus, as defined in Claim 1, wherein the lesion region input means (10) can input the positions of a plurality of lesion regions, and
wherein the link character insertion means can insert a plurality of link characters corresponding to the input positions of the plurality of lesion regions, respectively.

3. An image interpretation report generation apparatus, as defined in Claim 1 or 2, wherein the image interpretation report display means (30) displays, together with the image interpretation report, an attachment image obtained by reducing the medical image including the lesion region corresponding to the link character.

4. An image interpretation report generation apparatus, as defined in Claim 3, wherein the image interpretation report display means (30) displays a plurality of attachment images in the order of arrangement of the link characters in the image interpretation report.

5. An image interpretation report generation apparatus, as defined in any one of Claims 1 to 4, wherein the lesion region input means (10) can manually input the position of the lesion region.

6. An image interpretation report generation apparatus, as defined in any one of Claims 1 to 4, the apparatus further comprising:
a lesion name storage means (70) that stores lesion names representing a plurality of lesions; and
a lesion region detection means (80) that automatically detects the position of the lesion region in the medical image,
wherein the lesion region input means (10) inputs the position of the lesion region that has been automatically detected by the lesion region detection means (80), and
wherein the link character insertion means (20) selects, from the lesion name storage means (70), a lesion name representing the lesion present at the position of the lesion region that has been detected by the lesion region detection means (80), and inserts the lesion name.

7. An image interpretation report generation apparatus, as defined in anyone of Claims 2 to 6, wherein the index display means (60) displays the index, based on the selection of the link character in the image interpretation report, in such a manner that only the index corresponding to the selected link character is emphasized.

8. An image interpretation report generation apparatus, as defined in any one of Claims 2 to 7, wherein the link character insertion means (20) inserts the plurality of link characters in different colors from each other into the image interpretation report, and
wherein the index display means (60) displays the plurality of indices in the same colors as the colors of the link characters corresponding to the plurality of indices, respectively.

9. An image interpretation report generation method comprising the steps of:
obtaining and displaying a medical image;
generating and displaying an electronic image interpretation report of the medical image;
inputting the position of a lesion region in the medical image;
inserting, into the image interpretation report, a link character linked by a hyperlink to the medical image including the input position of the lesion region in such a manner that the link character is distinguishably displayed;
selecting the distinguishably displayed link character in the image interpretation report; and
displaying an index representing the position of the lesion region in the medical image based on the selection of the link character.

10. A computer-readable recording medium stored therein an image interpretation report generation program for causing a computer to function as the image interpretation report generation apparatus as defined in any one of claims 1 to 8.

## Patentansprüche

1. Vorrichtung zum Erstellen von Bildinterpretationsberichten, umfassend:
eine Medizinbild-Anzeigeeinrichtung (50), ausgebildet zum Gewinnen und Anzeigen eines medizinischen Bilds;
eine Bildinterpretationsbericht-Anzeigeeinrichtung (30), ausgebildet zum Erzeugen und Anzeigen eines elektronischen Bildinterpretationsberichts bezüglich des medizinischen Bilds; und
eine Verknüpfungszeichen-Auswähleinrichtung (40), die ein unterscheidbar angezeigtes Verknüpfungszeichen in dem Bildinterpretationsbericht auswählt;
**gekennzeichnet durch**
eine Läsionszonen-Eingabeeinrichtung (10), ausgebildet zum manuellen Eingeben der Stelle einer Läsionszone innerhalb des medizinischen Bilds;
eine Verknüpfungszeichen-Einfügeeinrichtung (20), ausgebildet, um in den Bildinterpretationsbericht das Verknüpfungszeichen einzufügen, das über einen Hyperlink mit dem medizinischen Bild verknüpft ist, einschließlich der eingegebenen Stelle der Läsionszone in der Weise, dass das Verknüpfungszeichen unterscheidbar angezeigt wird; und
eine Indexanzeigeeinrichtung (60), die basierend auf der Auswahl **durch** die Verknüpfungszeichen-Auswähleinrichtung einen Index anzeigt, der die Stelle der Läsionszone innerhalb des medizinischen Bilds repräsentiert.

2. Vorrichtung nach Anspruch 1, bei der die Läsionszonen-Eingabeeinrichtung (10) die Stellen mehrerer Läsionszonen eingeben kann, und
wobei die Verknüpfungszeichen-Einfügeeinrichtung mehrere Verknüfungszeichen entsprechend den angegebenen Positionen der mehreren Läsionszonen einfügen kann.

3. Vorrichtung nach Anspruch 1 oder 2, bei der die Interpretationsbericht-Anzeigeeinrichtung (30) zusammen mit dem Bildinterpretationsbericht ein Anhangbild anzeigt, welches erhalten wird durch Verkleinern des die Läsionszone entsprechend dem Verknüpfungszeichen enthaltenen medizinischen Bilds.

4. Vorrichtung nach Anspruch 3, bei der die Bildinterpretationsbericht-Anzeigeeinrichtung (30) mehrere Anhangbilder in der Reihenfolge der Anordnung der Verknüpfungszeichen innerhalb des Bildinterpretationsberichts anzeigt.

5. Vorrichtung nach einem der Ansprüche 1 bis 4, bei der die Läsionszonen-Eingabeeinrichtung (10) den Ort der Läsionszone manuell eingeben kann.

6. Vorrichtung nach einem der Ansprüche 1 bis 4, weiterhin umfassend:
eine Läsionsnamen-Speichereinrichtung (70), die für mehrere Läsionen repräsentative Läsionsnamen speichert; und
eine Läsionszonen-Nachweiseinrichtung (80), die automatisch die Stelle der Läsionszone in dem medizinischen Bild nachweist,
wobei die Läsionszonen-Eingabeeinrichtung (10) die Stelle der Läsionszone, die automatisch von der Läsionszonen-Nachweiseinrichtung (80) nachgewiesen wurde, eingibt, und
wobei die Verknüpfungszeichen-Einfügeeinrichtung (20) aus der Läsionsnamen-Speichereinrichtung (70) einen Läsionsnamen auswählt, der die an der von der Läsionszonen-Nachweiseinrichtung (80) nachgewiesenen Stelle der Läsionszone vorhandene Läsion repräsentiert, und den Läsionsnamen einfügt.

7. Vorrichtung nach einem der Ansprüche 2 bis 6, bei der die Indexanzeigeeinrichtung (60) den Index basierend auf der Auswahl des Verknüpfungszeichens in dem Bildinterpretationsbericht in der Weise anzeigt, dass nur der dem ausgezeichneten Verknüpfungszeichen ausgewählte Index hervorgehoben wird.

8. Vorrichtung nach einem der Ansprüche 2 bis 7, bei der die Verknüpfungszeichen-Einfügeeinrichtung (20) die mehreren Verknüpfungszeichen in voneinander verschiedenen Farben in den Bildinterpretationsbericht einfügt, und
wobei die Indexanzeigeeinrichtung (60) die mehreren in Indizes in den gleichen Farben wie den Farben der den mehreren Indizes entsprechenden Verknüpfungszeichen anzeigt.

9. Verfahren zum Erstellen von Bildinterpretationsberichten, umfassend folgende Schritte:
Gewinnen und Anzeigen des medizinischen Bilds;
Erzeugen und Anzeigen eines elektronischen Bildinterpretationsberichts des medizinischen Bilds;
Eingeben der Stelle einer Läsionszone innerhalb des medizinischen Bilds;
Einfügen eines durch einen Hyperlink mit dem medizinischen Bild verknüpften Verknüpfungszeichens einschließlich der eingegebenen Stelle der Läsionszone in den Bildinterpretationsbericht in der Weise, dass das Verknüpfungszeichen in unterscheidbarer Weise angezeigt wird;
Auswählen des unterscheidbar angezeigten Verknüpfungszeichens innerhalb des Bildinterpretationsberichts; und
Anzeigen eines Index', der die Stelle der Läsionszone innerhalb des medizinischen Bilds repräsentiert, basierend auf der Auswahl des Verknüpfungszeichens.

10. Computerlesbares Aufzeichnungsmedium mit einem darin gespeicherten Programm zum Erstellen eines Bildinterpretationsberichts, um einen Computer zu veranlassen, als Vorrichtung zum Erstellen eines Bildinterpretationsberichts gemäß einem der Ansprüche 1 bis 8 zu fungieren.

## Revendications

1. Appareil de génération de rapports d'interprétation d'image, comprenant :
un moyen d'affichage d'image médicale (50) apte à obtenir et afficher une image médicale ;
un moyen d'affichage de rapport d'interprétation d'image (30) apte à générer et afficher un rapport d'interprétation d'image électronique de l'image médicale, et
un moyen de sélection de caractère de liaison (40) qui sélectionne un caractère de liaison affiché de manière distinctive dans le rapport d'interprétation d'image,
**caractérisé par**
un moyen d'entrée de région de lésion (10) apte à entrer manuellement la position d'une région de lésion dans l'image médicale ;
un moyen d'insertion de caractère de liaison (20) apte à insérer, dans le rapport d'interprétation d'image, le caractère de liaison relié par un lien hypertexte à l'image médicale incluant la position d'entrée de la région de lésion, de manière à ce que le caractère de liaison soit affiché de manière distinctive, et
un moyen d'affichage d'index (60) qui affiche, sur la base de la sélection par le moyen de sélection de caractère de liaison, un index représentant la position de la région de lésion dans l'image médicale.

2. Appareil de génération de rapports d'interprétation d'image selon la revendication 1, dans lequel le moyen d'entrée de région de lésion (10) peut entrer les positions d'une pluralité de régions de lésion, et
dans lequel le moyen d'insertion de caractère de liaison peut insérer une pluralité de caractères de liaison correspondant respectivement aux positions d'entrée de la pluralité de régions de lésion.

3. Appareil de génération de rapports d'interprétation d'image selon la revendication 1 ou 2, dans lequel le moyen d'affichage de rapport d'interprétation d'image (30) affiche, conjointement au rapport d'interprétation d'image, une image de pièce jointe obtenue en réduisant l'image médicale incluant la région de lésion correspondant au caractère de liaison.

4. Appareil de génération de rapports d'interprétation d'image selon la revendication 3, dans lequel le moyen d'affichage de rapport d'interprétation d'image (30) affiche une pluralité d'images de pièces jointes dans l'ordre d'agencement des caractères de liaison dans le rapport d'interprétation d'image.

5. Appareil de génération de rapports d'interprétation d'image selon l'une quelconque des revendications 1 à 4, dans lequel le moyen d'entrée de région de lésion (10) peut entrer manuellement la position de la région de lésion.

6. Appareil de génération de rapports d'interprétation d'image selon l'une quelconque des revendications 1 à 4, l'appareil comprenant en outre :
un moyen de stockage de nom de lésion (70) qui stocke des noms de lésion représentant une pluralité de lésions, et
un moyen de détection de région de lésion (80) qui détecte automatiquement la position de la région de lésion dans l'image médicale,
dans lequel le moyen d'entrée de région de lésion (10) entre la position de la région de lésion qui a été automatiquement détectée par le moyen de détection de région de lésion (80), et
dans lequel le moyen d'insertion de caractère de liaison (20) sélectionne, à partir du moyen de stockage de nom de lésion (70), un nom de lésion représentant la lésion présente au niveau de la position de la région de lésion qui a été détectée par le moyen de détection de région de lésion (80), et insère le nom de lésion.

7. Appareil de génération de rapports d'interprétation d'image selon l'une quelconque des revendications 2 à 6, dans lequel le moyen d'affichage d'index (60) affiche l'index, sur la base de la sélection du caractère de liaison dans le rapport d'interprétation d'image, de manière à ce que seul l'index correspondant au caractère de liaison sélectionné soit mis en valeur.

8. Appareil de génération de rapports d'interprétation d'image selon l'une quelconque des revendications 2 à 7, dans lequel le moyen d'insertion de caractère de liaison (20) insère la pluralité de caractères de liaison dans des couleurs différentes entre les uns et les autres dans le rapport d'interprétation d'image, et
dans lequel le moyen d'affichage d'index (60) affiche la pluralité d'index dans les mêmes couleurs que les couleurs des caractères de liaison correspondant respectivement à la pluralité d'index.

9. Procédé de génération de rapports d'interprétation d'image comprenant les étapes consistant à :
obtenir et afficher une image médicale ;
générer et afficher un rapport d'interprétation d'image électronique de l'image médicale ;
entrer la position d'une région de lésion dans l'image médicale ;
insérer, dans le rapport d'interprétation d'image, un caractère de liaison relié par un lien hypertexte à l'image médicale incluant la position d'entrée de la région de lésion, de manière à ce que le caractère de liaison soit affiché de manière distinctive ;
sélectionner le caractère de liaison affiché de manière distinctive dans le rapport d'interprétation d'image, et
afficher un index représentant la position de la région de lésion dans l'image médicale sur la base de la sélection du caractère de liaison.

10. Support d'enregistrement pouvant être lu par ordinateur à l'intérieur duquel est stocké un programme de génération de rapports d'interprétation d'image destiné à amener un ordinateur à fonctionner comme l'appareil de génération de rapports d'interprétation d'image selon l'une quelconque des revendications 1 à 8.
